# EUROPEAN PATENT APPLICATION

(11) **EP 2 907 376 A1**
(43) Date of publication of application: **19.08.2015**
(21) Application number: 14305193.6
(22) Date of filing: 14.02.2014
(51) Int. Cl.: A01H 5/10, C12N 15/82

(54) **Method for plant improvement**

(71) Applicant: Biogemma, 75001 Paris (FR)
(72) Inventor: The designation of the inventor has not yet been filed
(74) Representative: Flesselles, Bruno F.G.

(57) **Abstract**

The invention relates to the field of plant improvement, in particular of the improvement of yield for plants, by using a transgene containing an endosperm promoter driving expression of a class A HsF protein.

## Description

The invention disclosed herein provides a method for producing transgenic plant with increased yield compared to non transgenic control plants. The present invention further relates to a nucleic construct increasing yield of the transgenic plants, and cell, seed and plant comprising the nucleic construct and method of producing and using such cell, seed and plant.

In agriculture, yield is the amount of product harvested from a given acreage (eg weight of seeds per unit area). It is often expressed in metric quintals (1 q = 100 kg) per hectare in the case of cereals. It is becoming increasingly important to improve the yield of seed crops to feed an expanding population and, more recently, for biofuel production. One strategy to increase the yield is to increase the seed size, provided that there is not a concomitant decrease in seed number.

One important issue to be achieved in transgenic crop is obtaining plants capable of maintaining or increasing yield under stress conditions compared to normal conditions. Stress conditions can correspond for example to abiotic stress like light stress, extreme temperatures (heat, cold and freezing), drought (lack of precipitations) or soil contamination by salt. All these environmental stresses can more or less impair plant development growth and ultimately yield.

Drought stress, or water deficit, occurs when water supply in the soil is reduced and/or water loss by transpiration or evaporation occurs continuously. When drought stress intensity is strong, it is called dessication. In field conditions, drought stress can also be combined to heat stress.

Determination of the molecular and genetic basis of the plant response to stress and notably drought stress is an important challenge for crop development.

Heat Shock transcription factors (Hsfs) genes are known to encode for heat shock transcription factors involved in the regulation of the expression of heat shock proteins (HSPs) which are essential in plant response to damage stress. Hsfs genes have been isolated from various different plant species and amongst the same specie: 21 genes are identified in *Arabidopsis thaliana,* 23 from *Oryza sativa* and 25 from Zea *mays* (Lin et al, BMC genomics 2011, 12:76). Hsfs genes structure and function are highly conserved in plant kingdom. One of the most conserved structures are the DNA Binding Domain (DBD and the oligomerization domains (HR-A/B).

In plants, Hsfs were long thought to belong to 2 classes, *ie,* class A and class B, but most recent reports indicate that there are three classes of plant Hsfs (classes A, B and C), based on peculiarities in the flexible linker and the HR-A/B domains (Nover et al, Cell Stress & Chaperones (2001) 6 (3), 177-189 and Miller and Mittler, Annals of Botany 98: 279-288, 2006). The HR-A/B region of class B Hsfs are similar to all non-plant Hsfs, whereas all class A and class C Hsfs have an extended HR-A/B region due to an insertion of 21 (class A) and 7 (class C) amino acid residues between the A and B parts

Class A HsFs, that comprise the largest group, further contain a C-terminal activation domain (CTAD), that is the least conserved in sequence and size (Nover et al., 2001). The function of the class A Hsfs as transcriptional activators depends on a short AHA motif found in the CTAD, enriched with aromatic, large hydrophobic and acidic amino acids (Nover and Scharf, 1997; Doring et al., 2000). AHA domains are crucial for the interaction of Hsfs with the transcriptional machinery, as was shown by pull-down experiments and functional analysis of class A Hsfs (Yuan and Gurley, 2000; Kotak et al., 2004). Another hydrophobic repeat (HR-C) is found at the C-terminal domain (Nover et al., 2001). This region is conserved among animal Hsfs but it is poorly conserved in yeast and plant Hsfs (Wu, 1995). The NES is a leucine-rich export signal found at the C-terminus of the CTAD.

Most of the Hsfs are expressed in many different tissues or in specific tissues. For example, in *Arabidopsis,* comparative study of transcripts abundance shows that Hsf3 (or HsfA1b) is expressed in all tissues and HsfA9 gene is exclusively expressed in seeds. (Miller and Mittler, Annals of Botany 98:279-288, 2006).

Tomato HsfA1 gene is one of the most well-known Hsfs gene involved in heat tolerance. HsfA1 is identified as a master regulator of induced thermotolerance in tomato. Mishra et al (Genes and Dev,16:1555-1567, 2002) have obtained a tomato plant constitutively overexpressing HSFA1 protein 10 fold compared to wild type plant not transformed. They have shown that these plants are more tolerant to different heat stress treatments compared to the wild type plants and co-suppressed HsfA1 lines.

US 8,445,747 describes the overexpression under a strong promoter of heat shock protein in order to increase water productivity and water use efficiency in plant. Examples show the overexpression in Arabidopsis plant of HSF3 protein under a strong promoter CaMV35S. The transgenic and wild type plants are grown in drought and well watered conditions. In drought conditions, plants are not watered for 1-, 11 days to 2 weeks then rewatered until seed set. Transgenic plants overexpressing HSF3 have a higher seed yield in well watered conditions and after water deficit stress. Seed yield is assessed by measuring seed biomass or number of seeds per plant.

Bechtold et al have shown that overexpression of Hsf3/HsfA1 b in oil seed rape increases harvest index and seed yield (J. exp. Bot., 64-11 pp3467-3481, 2013)

WO 2011/061656 and US 20060150283 describe thousands of protein sequences as being involved in yield increase under normal or stress conditions. The sequence can be overexpressed or inhibited. A long list of different potential promoters that may be used is also given. Notably, it is suggested to use seed specific promoters. Table VIIIA shows that SEQ ID N°8206, encoding for an HSF2 protein in Arabidopsis, is used to transform an *Arabidopsis* plant by overexpression in cytoplasm, i.e. through non targeted expression. In this case, the vectors used are listed in table VII and they all comprise constitutive promoters. The result shows that *Arabidopsis* plants expressing HSF2 with a constitutive promoter harbor an increased biomass production (median weight) when plants are grown under limited nitrogen supply.

The complexity of the plant response is such that, as of today, no transgenic plant product harboring improved yield has been commercialized.

Consequently, there is a need of developing plants, notably monocotyledons, with maintained or improved yield capacity measured in field conditions under normal or drought stress conditions.

### SUMMARY OF THE INVENTION

The invention disclosed herein provides a method for producing a monocotyledon plant with increased yield in normal and/or drought conditions as compared to a wild type not transformed plant. The method comprises the step of transforming a plant with a gene coding for a HsF protein of class A, under the control of a promoter that is functional (i.e. that is capable of driving expression of the gene) in the endosperm of said monocotyledon plant. In a specific embodiment, said promoter is not the natural promoter of said Hsf gene (ie is a heterologous promoter with regards to said Hsf gene).

Spatial and temporal control is often important in driving gene expression in plants in order to obtain the expression of a specific trait. Gene can be expressed in specific part of the seeds by using specific promoters

The kernel consists of a number of parts, some derived from maternal tissue and others from the fertilization process. In maize, the kernel maternally inherits a number of tissues, including a protective, surrounding pericarp and a pedicel. The pedicel is a short stalk-like tissue which attaches the kernel to the cob and provides nutrient transfer from maternal tissue into the kernel.

The kernel also contains tissues resulting from the fertilization activities, including the new embryo as well as the endosperm. The embryo is the miniature progenitor of the next generation, and contains cells for root and shoot growth of a new, young maize plant. It is also one tissue in which oils and proteins are stored in the kernel. The endosperm functions more as a nutritive tissue and provides the energy in the form of stored starch, proteins and oil, needed for the germination and initial growth of the embryo.

The endosperm, which forms most of the volume and weight of the kernel, can be divided into three parts: starchy endosperm, aleurone layer and basal transfer layer.

The role of the starchy endosperm is to provide the nutrients to the seed. It is thus mainly composed by cells filled with reserves, such as starch granules, or protein bodies. These cells die during dessication of the seed and this compartment is thus composed of dead cells in mature seeds.

The aleurone layer is a maternal one-celled layer of cells in contact with the pericarp. Basically, these cells accumulate lipids and storage proteins. The cells remain active until maturation and play an important role during germination.

The basal endosperm transfer layer (BETL) cells are in contact with the pedicel at the base of the endosperm. They transport the nutrients from the mother plant to the embryo surrounding region, as direct transport is not possible since vascular tissue does not extend beyond the pedicel.

Multiple promoters functional in the endosperm have been described in the art, for various plants.

As a matter of illustration, one can cite the promoters described in WO 2010147825 A1, or the PzsS3a promoter, which is endosperm specific in maize (Yu-feng et al, Biotechnology letters, 2011, vol. 33, no7, pp. 1465-1471), the alpha globulin promoter from rice, the oat globulin promoter, the rice or wheat glutelin promoter, blz2, rice transcription factor RISBZ1..

For expression in aleurone, one can cite the promoters described by Hwang et al (Plant cell reports, 2001, vol. 20, no7, pp. 647-654) or Le Qing Qu et al (Journal of experimental botany, 2008, vol. 59, no9, pp. 2417-2424). One can also cite the pBETL9-like promoter (Gomez et al, 2009Plant Cell. 2009 July; 21(7): 2022-2035).

For expression in the BETL, one can cite promoters such as BETL-1 (Hueros et al, Plant Cell, Vol. 7, 747-757, 1995), BETL-2 (WO 99/50427) or BETL-9 (described as sequence 18 from US20090307795).

Some promoters functional in the starchy endosperm are illustrated in Wiley et al ( Plant Molecular Biology, May 2007, Volume 64, Issue 1-2, pp 125-136), Kawakatsu et al (Journal of experimental botany, 2008, vol. 59, no15, pp. 4233-4245), Lamacchia et al (Journal of experimental botany, 2001, vol. 52, no355, pp. 243-250) or Lovegrove et al (Plant physiology, 2013, vol. 163, no1, pp. 95-107) who describe use of a starchy endosperm-specific promoter for making transgenic wheat lines.

Other endosperm promoters are described in US 8,552,256, US 8,466,342, US 8,227,664 (see in particular Table 1 of this document), or US 7,847,160.

A particular promoter that can be used in the context of the invention is the HMWG promoter (High Molecular Weight Glutenin) of wheat (Anderson OD et al, NUCLEIC ACIDS RESEARCH, vol. 17, 1989, pages 461-462, Colot et al, Mol Gen Genet. 1989 Mar;216(1):81-90.

A sequence for this promoter is described as SEQ ID N° 8.

Other promoters active or even specific of the starchy endosperm are for example the waxy, zein or bronze promoters of maize (Russell et Fromm Transgenic Research, 6 157-168 (1997)).

The invention thus relates to a nucleic acid construct comprising
a) a promoter functional in the endosperm, operably linked to
b) a nucleic acid coding for a class A Heat shock transcription factors (HSF-A) protein.

Said nucleic acid may be called an "expression cassette".

Some promoters functional in the endosperm have described above. Other promoters can easily be used and identified by the person skilled in the art.

A promoter "functional in a given tissue" of a plant is a promoter that allows expression of a nucleic acid sequence operatively linked to it in said given tissue of said plant.

In another embodiment, said promoter is predominantly functional in the endosperm, i.e. said promoter can be active in other tissues than the endosperm, but the principal expression of a nucleic acid sequence encoding a protein operatively linked to it is in the endosperm. This can be verified, using various techniques known by the person skilled in the art, such as quantification of the RNA expression of said nucleic acid sequence in various tissues by Northern blot, or of the protein expression in various tissues by Western blot.

The promoter can be specific to the endosperm (or to a tissue of the endosperm). A promoter specific to a given tissue is active exclusively in said tissue, i.e. it is not possible to detect expression of a nucleic acid sequence encoding a protein operatively linked to it in other tissues than said given tissue.

In a specific embodiment, said promoter functional in the endosperm is functional in the BETL. In this embodiment, said promoter may be predominantly functional in the BETL or specific to the BETL.

In another embodiment, said promoter active in the endosperm is functional in the starchy endosperm. In this embodiment, said promoter may be predominantly functional in the starchy endosperm or specific to the starchy endosperm.

In another embodiment, said promoter active in the endosperm is functional in the aleurone. In this embodiment, said promoter may be predominantly functional in the aleurone or specific to the aleurone.

All properties that apply to the promoter functional in the endosperm are also intended to apply when the promoter is functional in the aleurone, in the starchy endosperm or in the BETL.

A preferred promoter is the HMWG promoter, a representative sequence of which is SEQ ID N° 8. It is to be noted that, although SEQ ID N° 8 is herein used to define the HMWG promoter, a sequence that is at least 90 % identical, preferably at least 95 % identical, more preferably at least 96 % identical, more preferably at least 97 % identical, more preferably at least 98 % identical, more preferably at least 99 % identical, more preferably at least 99.5 % identical, more preferably at least 99.7 % identical, more 15 preferably at least 99.9 % identical, more preferably at least 99.95 % identical to SEQ ID N° 8 can also be considered as being a HMWG promoter. This is because it is known that it is possible to change or delete (especially in the 5' end) a few nucleic acids in a promoter without modifying its pattern of expression. On can, in particular, cite the sequences that can be isolated from the GenBank accession numbers X12928.5, KC820631.1, DQ208971.1 or X03346.1, or the promoters that can be isolated from plants of the *Aegilops* (such as AY611720.1) the *Secale* (such as GU373813.1) the *Lophopyrum* (such as KC478930.1) or the *Heteranthelium* (such as EU074239.1) species or from *Triticum turgidum* (such as AY848709.1) *Triticum durum* (such as JQ689002.1) Sequences of particular interest are the ones that also present an e-value that is lower than 1 x e-150 when performing a blastn with SEQ ID N° 8 as a query, and the following parameters: sequences
Expect threshold: 10
Word size: 28
Max matches in a query range: 0
Scoring Parameters Match/Mismatch Scores: 1,-2
Gap Costs: Linear

All these sequences are designated as HMWG promoter, SEQ ID N° 8 being a specific sequence of this generic family, the sequences having the accession number as indicated above being other species of this genus.

Such sequences can easily be checked for their ability to induce expression of a gene in the endosperm, and in particular in the starchy endosperm. Comparing the patterns of expression of a modified promoter and of the promoter depicted by SEQ ID N° 8 can be performed using any reporter gene known in the art, such as the luciferase, GUS or GFP genes.

As intended herein, a "class A Hsf protein" is intended to designate a protein as classified as such in Nover et al, Cell Stress & Chaperones (2001) 6 (3), 177-189 and Miller and Mittler, Annals of Botany 98: 279-288, 2006. This protein thus contains an extended HR-A/B region containing an insertion of 21 amino acid residues between the A and B parts. This protein further contains a C-terminal activation domain (CTAD), which presents a AHA motif in the CTAD, enriched with aromatic, large hydrophobic and acidic amino acids. The class A Hsf further contains another hydrophobic repeat (HR-C) found at the C-terminal domain.

Class A HsF proteins are well known by the person skilled in the art. Among preferred proteins that can be used in the nucleic acid consztruct described above, one can cite the proteins listed in Figure 2 of Miller and Mittler (2006).

In particular, one can use the *Arabidopsis* protein, the sequence of an allele of which is depicted by SEQ ID N° 1.

One can also use a maize protein, the sequence of an allele of which is represented by SEQ ID N° 2.

One can also use a rice protein, the sequence of an allele of which is represented by SEQ ID N° 3.

One can also use a rice protein, the sequence of an allele of which is represented by SEQ ID N° 4.

One can also use a soybean protein, the sequence of an allele of which is represented by SEQ ID N° 5.

One can also use a sorghum protein, the sequence of an allele of which is represented by SEQ ID N° 6.

One can also use a barley protein, the sequence of an allele of which is represented by SEQ ID N° 7.

It is also to be noted that the sequences SEQ ID N° 1 to SEQ ID N° 7 represent and exemplify alleles of class A HsF proteins in various plants. There exists different other alleles of this protein in said plants, and different other class A Hsf proteins in said plants or in other plants, which all possess the same activity than this protein, and can be used in the above-identified nucleic acid construct.

These proteins may be identified, by applying the BLASTP program (especially the BLASTP 2.2.29 program) (Altschul et al, (1997), Nucleic Acids Res. 25:3389-3402; Altschul et al, (2005) FEBS J. 272:5101-5109) to any of SEQ ID N° 1 to SEQ ID N° 7, using the following algorithm parameters:
Expected threshold : 10
Word size : 3
Max matches in a query range: 0
Matrix: BLOSUM62
Gap Costs: Existence 11, Extension 1.
Compositional adjustments: Conditional compositional score matrix adjustment
No filter for low complexity regions

The proteins that can be used in the context of the above construct are preferably the ones, the sequence of which presents a Max score above 500, and a E value of 0.0 or less than 1e⁻¹⁷⁰, with at least one sequence chosen in the group consisting of SEQ ID N° 1, SEQ ID N° 2, SEQ ID N° 3, SEQ ID N° 4, SEQ ID N° 5, SEQ ID N° 6 and SEQ ID N° 7.

Their sequence would also preferably present an identity (as indicated by the BLAST2P software) equal or above 70%, preferably, equal or above 80% more preferably equal or above 90%, more preferably equal or above 92%, more preferably equal or above 93%, more preferably equal or above 94%, more preferably equal or above 95%, more preferably equal or above 96%, more preferably equal or above 97%, more preferably equal or above 98%, more preferably equal or above 99% more preferably equal or above 99.5%, more preferably equal or above 99.8% with SEQ ID N ° 1.

Their sequence would also preferably present an identity (as indicated by the BLAST2P software) equal or above 70%, preferably, equal or above 80% more preferably equal or above 90%, more preferably equal or above 92%, more preferably equal or above 93%, more preferably equal or above 94%, more preferably equal or above 95%, more preferably equal or above 96%, more preferably equal or above 97%, more preferably equal or above 98%, more preferably equal or above 99% more preferably equal or above 99.5%, more preferably equal or above 99.8% with SEQ ID N ° 2.

Their sequence would also preferably present an identity (as indicated by the BLAST2P software) equal or above 70%, preferably, equal or above 80% more preferably equal or above 90%, more preferably equal or above 92%, more preferably equal or above 93%, more preferably equal or above 94%, more preferably equal or above 95%, more preferably equal or above 96%, more preferably equal or above 97%, more preferably equal or above 98%, more preferably equal or above 99% more preferably equal or above 99.5%, more preferably equal or above 99.8% with SEQ ID N ° 3.

Their sequence would also preferably present an identity (as indicated by the BLAST2P software) equal or above 70%, preferably, equal or above 80% more preferably equal or above 90%, more preferably equal or above 92%, more preferably equal or above 93%, more preferably equal or above 94%, more preferably equal or above 95%, more preferably equal or above 96%, more preferably equal or above 97%, more preferably equal or above 98%, more preferably equal or above 99% more preferably equal or above 99.5%, more preferably equal or above 99.8% with SEQ ID N ° 4.

Their sequence would also preferably present an identity (as indicated by the BLAST2P software) equal or above 70%, preferably, equal or above 80% more preferably equal or above 90%, more preferably equal or above 92%, more preferably equal or above 93%, more preferably equal or above 94%, more preferably equal or above 95%, more preferably equal or above 96%, more preferably equal or above 97%, more preferably equal or above 98%, more preferably equal or above 99% more preferably equal or above 99.5%, more preferably equal or above 99.8% with SEQ ID N ° 5.

Their sequence would also preferably present an identity (as indicated by the BLAST2P software) equal or above 70%, preferably, equal or above 80% more preferably equal or above 90%, more preferably equal or above 92%, more preferably equal or above 93%, more preferably equal or above 94%, more preferably equal or above 95%, more preferably equal or above 96%, more preferably equal or above 97%, more preferably equal or above 98%, more preferably equal or above 99% more preferably equal or above 99.5%, more preferably equal or above 99.8% with SEQ ID N ° 6.

Their sequence would also preferably present an identity (as indicated by the BLAST2P software) equal or above 70%, preferably, equal or above 80% more preferably equal or above 90%, more preferably equal or above 92%, more preferably equal or above 93%, more preferably equal or above 94%, more preferably equal or above 95%, more preferably equal or above 96%, more preferably equal or above 97%, more preferably equal or above 98%, more preferably equal or above 99% more preferably equal or above 99.5%, more preferably equal or above 99.8% with SEQ ID N ° 7.

In a preferred embodiment, said nucleic acid comprises the HMWG promoter operably linked to the Arabidopsis class A Hsf depicted by SEQ ID N° 1.

In this embodiment, said nucleic acid may contain SEQ ID N° 9.

The invention also encompasses a vector containing the nucleic acid construct (expression cassette) of the invention.

A vector, such as a plasmid, can thus be used for transforming host cells. The construction of vectors for transformation of host cells is within the capability of one skilled in the art following standard techniques.

The decision as to whether to use a vector for transforming a cell, or which vector to use, is guided by the method of transformation selected, and by the host cell selected.

Where a naked nucleic acid introduction method is used, then the vector can be the minimal nucleic acid sequences necessary to confer the desired phenotype, without the need for additional sequences.

Possible vectors include the Ti plasmid vectors, shuttle vectors designed merely to maximally yield high numbers of copies, episomal vectors containing minimal sequences necessary for ultimate replication once transformation has occured, transposon vectors, including the possibility of RNA forms of the gene sequences. The selection of vectors and methods to construct them are commonly known to persons of ordinary skill in the art and are described in general technical references (Mullis, K B (1987), Methods in Enzymology).

For other transformation methods requiring a vector, selection of an appropriate vector is relatively simple, as the constraints are minimal. The apparent minimal traits of the vector are that the desired nucleic acid sequence be introduced in a relatively intact state. Thus, any vector which produces a plant carrying the introduced DNA sequence should be sufficient. Also, any vector which introduces a substantially intact RNA which can ultimately be converted into a stably maintained DNA sequence should be acceptable.

For transformation methods within a plant cell, one can cite methods of direct transfer of genes such as direct micro-injection into plant embryos, vacuum infiltration or electroporation, direct precipitation by means of PEG or the bombardment by gun of particules covered with the plasmidic DNA of interest.

It is preferred to transform the plant cell with a bacterial strain, in particular *Agrobacterium,* in particular *Agrobacterium tumefaciens.* In particular, it is possible to use the method described by Ishida et al. (Nature Biotechnology, 14, 745-750, 1996) for the transformation of monocotyledons.

However, any additional attached vector sequences which confer resistance to degradation of the nucleic acid fragment to be introduced, which assists in the process of genomic integration or provides a means to easily select for those cells or plants which are actually, in fact, transformed are advantageous and greatly decrease the difficulty of selecting useable transgenic plants.

The vector can exist, for example, in the form of a phage, a plasmid or a cosmid. The construction of such expression vectors for transformation is well known in the art and uses standard techniques. Mention may be made of the methods described by Sambrook et al. (1989).

For transforming bacteria, a vector is generally defined as being a nucleic acid molecule that possesses elements that allows it to be maintained within said host cell (such as an origin of replication that works in this bacterial host cell).

The invention also encompasses a host cell containing the nucleic acid construct (expression cassette) as described above.

The decision as to whether to use a given host cell, or which host cell to use, is guided by the method of transformation.

The host cell can be any prokaryotic or eukaryotic cell. Any of a large number of available and well-known host cells may be used in the practice of this invention. The selection of a particular host is dependent upon a number of factors recognized by the art. These include, for example, compatibility with the chosen expression vector, bio-safety and costs. Useful hosts include bacteria such as *E. coli* sp. or *Agrobacterium.* A plant host cell, may be also used, notably an angiosperm plant cell, monocotyledon as dicotyledon plant cell, particularly a cereal or oily plant cell, selected in particular from the group consisting of maize, wheat, barley, rice, rape and sunflower, preferentially maize. Another preferred plant is wheat. Another preferred plant is rice. Another preferred plant is barley.

More particularly, the host cell used in carrying out the invention is *Agrobacterium tumefaciens,* according to the method described in the article of An et al., 1986, or *Agrobacterium rhizogenes,* according to the method described in the article of Jouanin et al., 1987.

In a specific embodiment, said expression cassette is stably integrated within the genome of said host cell. This embodiment is particularly interesting for plant host cells. Stable integration within the genome means that the expression cassette can be transmitted to the progeny of said host cell upon division.

The invention also encompasses a plant containing at least one cell containing the expression cassette as defined above, preferably stably integrated within its genome.

A part of a transgenic plant, in particular fruit, seed, grain or pollen, comprising such a cell or generated from such a cell is also encompassed by the invention.

It is reminded that a whole plant can be regenerated from a single transformed plant cell. Thus, in a further aspect the present invention provides transgenic plants (or parts of them) including the expression cassette as described above. The regeneration can be performed by known methods.

The seeds which grow by fertilization from this plant, also contain this transgene in their genome.

Said plant or part of a plant according to the invention can be a plant or a part of it from various species, notably an Angiosperm, Monocotyledons as Dicotyledons.

It is preferably a cereal or oily plant. As used herein, the term "oily plant" denotes a plant that is capable of producing oil, and preferably that is cultivated for oil production.

Said plant is preferably selected from the group consisting of maize, rice, wheat, barley, rape and sunflower. In a preferred embodiment, said plant is maize. In another preferred embodiment, said plant is wheat.

The invention thus relates in particular to a transgenic maize or a transgenic wheat, containing at least one cell comprising, stably integrated in its genome, the expression cassette as disclosed above.

In a specific embodiment, said plant, in particular said maize, comprises multiple cells containing, stably integrated in their genome, the expression cassette as described above. In this embodiment, it is possible that some cells of said plant do not contain the transgene.

Due to the use of an endosperm promoter, expression of the class A HsF protein is thus predominant in the endosperm, and especially in the aleurone, starchy endosperm and/or BETL. This may be observed by performing Northern blot on RNA obtained from different organs of the plant, and detecting an expression at least ten times higher in the endosperm than in other organs.

In a specific embodiment, said transgene (comprising the expression cassette as disclosed) is present in all cells of said plant, in particular said maize or wheat.

In another embodiment, the transgene is introduced within the plant cells such as being expressed transiently, or through a genetic construct not integrated in the genome. Thus, agro-infiltration or any other methods, such as injection or spray, are contemplated for transient expression.

Hybrid plants obtained by crossing plants according to the invention also form part of the invention, when they contain at least one cell containing the expression cassette of the invention.

Any plant as described above can contain one or more transgenes in addition to the cassette according to the invention. One may mention transgenes conferring male sterility, male fertility, resistance to a herbicide (notably glyphosate, glufosinate, imidazolinone, sulfonylurea, L-phosphinotricine, triazine, benzonitrile), resistance to insects (notably a transgene coding for a *Bacillus thuringiensis* toxin), tolerance to water stress. These plants can be obtained by crossing said plants of the invention with other plants containing said transgenes. Alternatively, plants can be co-transformed with an expression cassette containing several different transgenes, including the transgene of the invention.

As demonstrated in the examples, said transgenic plants comprising an expression cassette according to the invention present an increased yield as compared to control plants corresponding to non-transgenic plants not comprising said expression cassette.

Said increased yield may be observed in normal conditions or in stress conditions.

Increased yield in stress conditions (or stress tolerance) can be measured by the ability of the transgenic plant to maintain yield under stress conditions compared to normal conditions (which is considered to be achieved when the yield observed in stressed conditions is at least 90%, 94%, 95%, 96%, 97%, 98%, 99% or 100% of the yield obtained for the same plant in non-stressed (normal) conditions). It can also be measured by the ability of the transgenic plant to increase yield under stress conditions compared to control plants grown under stress conditions (at least 101%, 102%, 103%, 104%, 105%, 106%, 107%, 108%, 109%, 110%, 111%, 112%, 113%, 114%, 115%).

As intended herein, stress conditions comprise specific conditions that are applied to a plant at a specific development stage such as that they induce a yield decrease of at least 8%, 10%, preferentially at least 15% and more preferentially at least 20% between the control plants in normal and in stress conditions. As a matter of illustration, one can cite heat stress conditions that may be applied during the flowering stage (in particular for wheat) or hydric stress before the flowering stage or after the fertilization, (in particular during the grain filling stage for maize).

Consequently, the invention also relates to various methods of making or using the plants of the invention.

Therefore, the invention also relates to a method for obtaining a transgenic plant containing at least one cell comprising a transgene comprising the nucleic acid construct as described above, comprising the steps consisting of:
a) transforming at least a plant cell or plant tissue with a vector containing the nucleic acid construct according to the invention;
b) cultivating the cell(s) or plant tissue thus transformed so as to generate a transgenic plant containing at least a cell which contains, in its genome, at least the nucleic acid construct as disclosed above.

One can also cite a method for obtaining a plant containing a transgene, wherein said transgene comprises the nucleic acid construct as disclosed above, comprising the steps of
a) Performing the method as described above (transformation of plant cells and regeneration) in order to obtain a transgenic plant, wherein said transgene comprises said nucleic acid construct described above,
b) crossing said transgenic plant with a plant line which does not contain said transgene (the receiver plant line)
c) selecting, among the progeny, plants that contain said transgene and that have a good genome ratio with regard to said receiver plant line,
d) back-crossing said selected plants with said receiver plant line
e) repeating steps c) and d) if necessary until a line isogenic with said receiving line (and containing said transgene) is obtained,
f) optionally, performing self-fertilization in order to obtain a plant homozygotic for said transgene.

The selection of step c) is preferably performed, by genotyping using molecular markers (for example microsatellite markers), making it possible to define the contribution of each of the two parents to the progeny. One would thus select, in the progeny, plants carrying the transgene and having more markers from the receiver plant line than from the parent containing the transgene.

Plants (in particular maize or wheat) which possess the transgene, may also be selected from the progeny, in a conventional manner by molecular biology methods (such as PCR or Southern blotting).

Said generated plant can also be used in a selection (breeding) process for obtaining a plant with improved yield.

The invention thus also relates to a method for producing a plant that can be used in a selection (breeding) process or scheme for obtaining a plant with improved yield, comprising the step of transforming a plant cell with a vector according to the invention, and regenerating a transgenic plant which comprises at least one cell which contain the transgene comprising the expression cassette as described above.

The introgression of the transgene in a given plant is in particular carried out by selection, according to methods known in the art (crossing and self-pollination). The plants are in particular selected using molecular markers.

The principle is recalled below:
A series of back crosses are performed between the elite line (in which one wishes to introduce the determinant) and a line that already carries said determinant (the donor line). During the back crosses, one can select individuals carrying the determinant and having recombined the smallest fragment from the donor line around the determinant. Specifically, by virtue of molecular markers, the individuals having, for the markers closest to the determinant, the genotype of the elite line are selected.

In addition, it is also possible to accelerate the return to the elite parent by virtue of the molecular markers distributed over the entire genome. At each back cross, the individuals having the most fragments derived from the recurrent elite parent will be chosen.

Selection is important as it is often preferable to sow and harvest plant lines that have been optimized, in particular for the location in which they are cultured. Consequently, one needs to introduce the transgene in said adapted lines having otherwise agronomic quality characteristics.

The invention also relates to a method for growing a plant, comprising the step of sowing a plant seed, wherein said plant seed contains the nucleic acid construct as described above, and growing plants from this sowed seed.

The invention also relates to a method for increasing plant yield under normal conditions, comprising the step of sowing plant seeds, wherein said plant seeds contain the expression cassette of the invention and growing plants from these sowed seeds, and wherein the yield obtained from said grown plants is increased as compared to the yield obtained from isogenic plants grown from seeds which do not contain said expression cassette

The invention also relates to a method for increasing or maintaining plant yield under stressed conditions, comprising the step of sowing plant seeds, wherein said plant seeds contain the nucleic acid construct as described above, and growing plants from these sowed seeds, wherein at least part of the growing phase is made under stress conditions, and wherein the yield obtained from said grown plants is increased as compared to the yield obtained from plants grown from seeds which do not contain said nucleic acid construct or the yield obtained from said grown plants is maintained as compared to the yield obtained from plants containing said nucleic acid construct and grown in normal conditions.

The invention also relates to a method of growing plants, comprising the step of sowing seeds containing the nucleic acid construct as described above, , and growing plants from the sowed seeds.

The invention may also comprise the step of harvesting said plants.

The invention also relates to a method for harvesting plants comprising the step of harvesting plants of the invention.

In particular, in the methods as described above, a hydric stress is applied to the plants during their growth.

A method for selecting (i.e. screening, identifying) a plant that can be used in a selection (breeding) process for obtaining a plant with improved yield, which comprises the step of selecting, in a population of plants, the plants containing the expression cassette as described above, is also part of the invention.

A breeding process for obtaining a plant with improved yield is performed as follows: the yield of a plurality of plants gives the reference yield level which is to be improved. The plant with improved yield is obtained, when the yield observed after sowing and harvesting said plant is higher than the yield of reference. Said plant with improved yield is obtained by known methods in the art, by crossing, back-crossing and stabilizing plants which present a yield

In a specific embodiment, the selection is performed through the use of a marker that is specific to the transgene. In this embodiment, the selection step is thus preferably preceded by a step comprising genotyping said population of cereals.

In a specific embodiment, the selection step is preceded by a step comprising extracting the RNA from the individuals in said population.

In a specific embodiment, the selection step is preceded by a step comprising extracting proteins from the individuals in said population.

In a specific embodiment, said population is the progeny obtained from crossing a transgenic plant, wherein said transgene comprises the expression cassette as described above, with a plant line which does not contain said transgene (the receiver plant line).

A method for identifying a plant with improved yield, which comprises the step of identifying, in a population of plants, the plants containing the expression cassette as described above, is also part of the invention. Improved yield is determined after comparison with a isogenic plant which does not contain the expression cassette.

In a specific embodiment, the identification is performed through the use of a marker that is specific to the transgene. In this embodiment, the identification step is thus preferably preceded by a step comprising genotyping said population of cereals.

In a specific embodiment, the identification step is preceded by a step comprising extracting the RNA from the individuals in said population.

In a specific embodiment, the identification step is preceded by a step comprising extracting proteins from the individuals in said population.

In a specific embodiment, said population is the progeny obtained from crossing a transgenic plant, wherein said transgene comprises the expression cassette as described above, with a plant line which does not contain said transgene (the receiver plant line).

The invention also relates to a method for obtaining a hybrid plant, wherein said hybrid plant contains the expression cassette as described above stably integrated within its genome. Said method comprises the step of crossing a first homozygous line, which contains said expression cassette stably integrated within its genome, with a second homozygous line.

This plant can be homozygous (if each homozygous parent has the expression cassette as described above stably integrated within its genome) or heterozygous for the transgene present on said expression cassette.

In a preferred embodiment, the methods are applied to a cereal (in particular, rice, maize, wheat, barley). It is preferred when said plant is maize or wheat.

### DESCRIPTION OF THE FIGURE

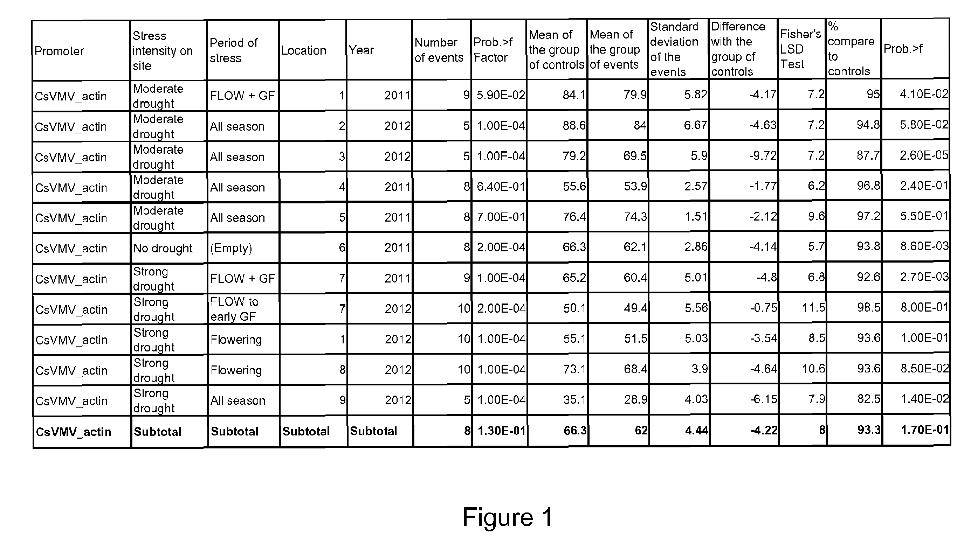

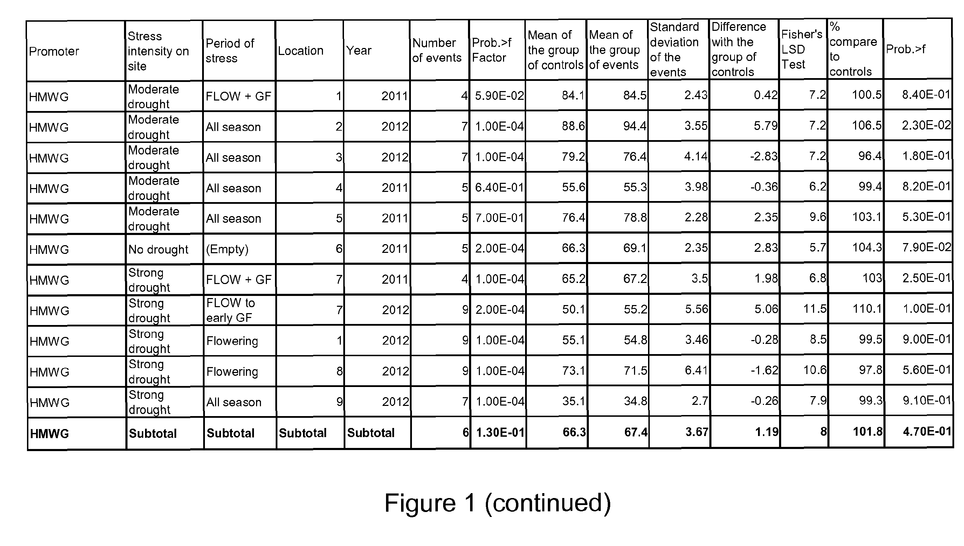

Figure 1 represents the results obtained for different events bearing the AtHSF3 coding sequence operably linked to either the CsVMV promoter + the actin intron (constitutive promoter) or the HMWG promoter (promoter active in the endosperm). Promoter: promoter used; Stress intensity on site, as observed; Period of stress: FLOW = flowering, GF = grain filling; Mean of the group of controls: mean yield observed for the control plants; Mean of the group of events: mean yield observed for the transgenic plants.

### Example 1 - Cloning of AtHSF3 downstream the CsVMV promoter and downstream the promoter HMWG and transformation

The AtHSF3 coding sequence (AT5G16820) was codon optimized for maize expression by a gene synthesis service provider (Genscript) and cloned into the pENTR4 vector (Invitrogen). The AtHSF3 sequence was then cloned via a GATEWAY LR reaction, between a CsVMV promoter (Verdaguer et al (1996)) plus a rice actin intron (McElroy et al 1990), and an Arabidopsis Sac66 polyadenylation sequence (Jenkins et al (1999)), into the destination binary plasmid pBIOS886 forming pBIOS1695. (The binary vector pBIOS886 is a derivative of pSB12 (Komari et al.(1996)) containing a pActin+actin intron-selectable marker- nos polyA chimeric gene for selection of maize transformants and a GATEWAY R1 R2 cassette between a CsVMV promoter+ actin intron and a Sac66 polyadenylation sequence). Similarly AtHSF3 was linked to the wheat HMWG promoter by performing a GATEWAY LR reaction between pENTR4-AtHSF3 and the destination binary plasmid pBIOS976 forming pBIOS1698. (The binary vector pBIOS976 is a derivative of pSB12 (Komari et al.(1996)) containing a pActin+actin intron-selectable marker- nos polyA chimeric gene for selection of maize transformants and a GATEWAY R1 R2 cassette between a HMWG promoter and a Sac66 polyadenylation sequence).
pBIOS1695 and pBIOS1698 were transferred into agrobacteria LBA4404 (pSB1) according to Komari et al (1996) forming respectively strain T 01748 and strain T 01751. Maize cultivar A188 was transformed with these agrobacterial strains essentially as described by Ishida et al (1996).
Analysis of the pCsVMV - AtHSF3 and pHMWG - AtHSF3 transformed maize plants indicated that some plants overexpressed AtHSF3.

### Example 2 - Maize field trials

Field trials show that seed yield and the stability of yield is improved when HSF3 is expressed from the endosperm-specific promoter HMWG.

### A - Field trials

Hybrids with a tester line were obtained from T3 plants issued from the AtHSF3 transgenic maize line (pro CsVMV + AtHSF3 + Sac66 term or proHMWG + AtHSF3 + Sac66 term) chosen according to example 1.
The transformants (T0) plant was first crossed with the A188 line thereby producing T1 plants. T1 plants were then self-pollinated twice, producing T3 plants which are homozygous lines containing the transgene. These T3 plants were then crossed with the tester line thereby leading to a hybrid. This hybrid is at a T4 level with regards to the transformation step and is heterozygous for the transgene. These hybrid plants are used in field experiments.

Control hybrids are obtained as follows:
Control Equiv corresponds to a cross between a A188 line (the line used for transformation) and the tester line.
Control Null segregant correspond to a cross between a null segregant (isolated after the second self-pollination of the T1 plants) and the tester line. Said null segregant is a homozygous line which does not bear the transgene. Although the null segregant theoretically presents the same genome as A188, it has undergone in vitro culture (via the steps of callus differentiation and regeneration) and may thus present mutations (either genetic or epigenetic) with regards to a A188 line that has not undergone *in vitro* culture.
These two control lines are used to avoid any effect that could be due to mutations (genetic or epigenetic) coming from in vitro culture.

Yield was calculated as follows:
During harvest, grain weight and grain moisture are measured using on-board equipment on the combine harvester.
Grain weight is then normalized to moisture at 15 %, using the following formula: Normalized grain weight = measured grain weight x (100 - measured moisture (as a percentage)) / 85 (which is 100 - normalized moisture at 15 %).
As an example, if the measured grain moisture is 25 %, the normalized grain weight will be: normalized grain weight = measured grain weight x 75 / 85.
Yield is then expressed in a conventional unit (such as quintal per hectare).

### B- Experimental design:

Field trials were conducted in 2011 and in 2012 on different locations.

In 2011, plants were sown between May 18 and June 16 2011., Harvest was between the 15^{th} of September and the 15th of October, 2011.

In 2012, plants were sown between May 18 and June 1st 2012., Harvest was between the 17^{th} of September and the 9^{th} of October, 2012.

The experimental block comprises 5 to 6 replicates. The experimental design was Randomized complete block or Lattice in both stressed locations and non-stressed locations. Each replicate comprised of two row plots with about up to 70 plants per plot at a density of 75 000 plants/ha.

Controls were used present in this experiment as described above (null segregant and a control equivalent (A188 crossed with the tester line).

A drought stressed location is a location where the grain yield potential of the site has not been reached due to a drought stress.

A non-stressed location is a location where the grain yield potential has been reached by a commercial hybrid variety.

The drought stress intensity is evaluated by measuring the yield lost between the drought stress treatment (WUE) and a reference treatment irrigated with an optimal amount of water, which is at least, equivalent to the maximum evapotranspiration (ETM) of the crop.

A yield lost of -30% is targeted with a common distribution of the drought location between -10% and -40% of yield.

A moderate drought stressed location is typically a location with a yield lost below -20%, a moderate stressed location below -30%.

The targeted growth stage period is typically from tasseling to R2 growth stage.

In a common drought location, the drought stress period can spread out from a period between V10 and R4 growth stage.

Figure 1 shows that expressing the class A HsF protein under the control of a constitutive promoter (CSVMV promoter) leds to a decrease in yield, in normal or stressed conditions, whereas use of an endosperm promoter (HMWG promoter) leads to a maintained or increased yield in normal or stressed conditions.

## Claims

1. A nucleic acid construct comprising a) a promoter functional in the endosperm, operably linked to b) a nucleic acid coding for a class A Heat shock transcription factors (HSF-A) protein.

2. The nucleic acid of claim 1, wherein said promoter functional in the endosperm is functional in the BETL.

3. The nucleic acid of claim 1, wherein said promoter active in the endosperm is functional in the starchy endosperm.

4. The nucleic acid of claim 1, wherein said promoter active in the endosperm is functional in the aleurone.

5. The nucleic acid of any one of claims 1 to 4, wherein the sequence of said HSF-A protein presents at least 70 or 80 or 90 or 95%, 96, 97, 98, 99, 99,5, 99,8% identity with at least one sequence chosen in the group consisting of SEQ ID N° 1, SEQ ID N° 2, SEQ ID N° 3, SEQ ID N° 4, SEQ ID N° 5, SEQ ID N° 6 and SEQ ID N° 7.

6. The nucleic acid of any one of claims 1 to 5, wherein an allele of said HSF-A protein is depicted by SEQ ID N° 1.

7. The nucleic acid construct of one any of claims 1 to 6, wherein said promoter is a HMWG promoter.

8. The nucleic acid construct of claim 7, wherein said promoter is the HMWG promoter as depicted in SEQ ID N° 8.

9. The nucleic acid construct of any one of claim 1 to 8, comprising SEQ ID N° 9.

10. A host cell containing the nucleic acid construct of any one of claims 1 to 9.

11. The host cell of claim 10, wherein said expression cassette is stably integrated within the genome of said host cell.

12. A transgenic plant, or a part of a transgenic plant comprising at least one cell according to claim 10 or 11.

13. The plant or part of a plant of claim 12, which is a cereal.

14. The plant or part of a plant of claim 13, wherein said plant is chosen among maize, wheat, barley and rice.

15. A method for increasing plant yield, comprising the step of sowing plant seeds, wherein said plant seeds contain the nucleic acid construct of any of claims 1 to 9 and growing plants from these sowed seeds, and wherein the yield obtained from said grown plants is increased as compared to the yield obtained from plants grown from seeds which do not contain the nucleic acid construct of any one of claims 1 to 9.

16. A method for increasing or maintaining plant yield under stressed conditions, comprising the step of sowing plant seeds, wherein said plant seeds contain the nucleic acid construct of any one of claims 1 to 9 and growing plants from these sowed seeds, wherein the growing phase is made under stress conditions, and wherein the yield obtained from said grown plants is increased as compared to the yield obtained from plants grown from seeds which do not contain the nucleic acid construct of any one of claims 1 to 9 or the yield obtained from said grown plants is maintained as compared to the yield obtained from plants containing the nucleic acid construct of any one of claims 1 to 10 and grown in normal conditions.

17. A method for selecting a plant that can be used in a breeding process for obtaining a plant with improved yield comprising the step of selecting, in a population of plant, the plants containing the nucleic acid construct of any one of claims 1 to 9.

18. A method for identifying a plant with improved yield comprising the step of identifying, in a population of plant, the plants containing the nucleic acid construct of any one of claims 1 to 9.

19. The method of any one of claims 15 to 18, wherein said plant is a cereal.

20. The method of claim 19, wherein said plant is maize.

21. The method of claim 19, wherein said plant is wheat.


SEQUENCE LISTING

<110>  BIOGEMMA

<120>  METHOD FOR PLANT IMPROVEMENT

<130>  BRV 88- EP PRIO

<160>  9

<170>  PatentIn version 3.3

<210>  1
<211>  481
<212>  PRT
<213>  Arabidopsis thaliana

<400>  1

Met Glu Ser Val Pro Glu Ser Val Pro Ser Pro Asn Ser Asn Thr Pro
1               5                   10                  15

Ser Ile Pro Pro Pro Val Asn Ser Val Pro Pro Phe Leu Ser Lys Thr
            20                  25                  30

Tyr Asp Met Val Asp Asp Pro Leu Thr Asn Glu Val Val Ser Trp Ser
        35                  40                  45

Ser Gly Asn Asn Ser Phe Val Val Trp Ser Ala Pro Glu Phe Ser Lys
    50                  55                  60

Val Leu Leu Pro Lys Tyr Phe Lys His Asn Asn Phe Ser Ser Phe Val
65                  70                  75                  80

Arg Gln Leu Asn Thr Tyr Gly Phe Arg Lys Val Asp Pro Asp Arg Trp
                85                  90                  95

Glu Phe Ala Asn Glu Gly Phe Leu Arg Gly Arg Lys Gln Leu Leu Lys
            100                 105                 110

Ser Ile Val Arg Arg Lys Pro Ser His Val Gln Gln Asn Gln Gln Gln
        115                 120                 125

Thr Gln Val Gln Ser Ser Ser Val Gly Ala Cys Val Glu Val Gly Lys
    130                 135                 140

Phe Gly Ile Glu Glu Glu Val Glu Arg Leu Lys Arg Asp Lys Asn Val
145                 150                 155                 160

Leu Met Gln Glu Leu Val Arg Leu Arg Gln Gln Gln Gln Ala Thr Glu
                165                 170                 175

Asn Gln Leu Gln Asn Val Gly Gln Lys Val Gln Val Met Glu Gln Arg
            180                 185                 190

Gln Gln Gln Met Met Ser Phe Leu Ala Lys Ala Val Gln Ser Pro Gly
        195                 200                 205

Phe Leu Asn Gln Leu Val Gln Gln Asn Asn Asn Asp Gly Asn Arg Gln
    210                 215                 220

Ile Pro Gly Ser Asn Lys Lys Arg Arg Leu Pro Val Asp Glu Gln Glu
225                 230                 235                 240

Asn Arg Gly Asp Asn Val Ala Asn Gly Leu Asn Arg Gln Ile Val Arg
                245                 250                 255

Tyr Gln Pro Ser Ile Asn Glu Ala Ala Gln Asn Met Leu Arg Gln Phe
            260                 265                 270

Leu Asn Thr Ser Thr Ser Pro Arg Tyr Glu Ser Val Ser Asn Asn Pro
        275                 280                 285

Asp Ser Phe Leu Leu Gly Asp Val Pro Ser Ser Thr Ser Val Asp Asn
    290                 295                 300

Gly Asn Pro Ser Ser Arg Val Ser Gly Val Thr Leu Ala Glu Phe Ser
305                 310                 315                 320

Pro Asn Thr Val Gln Ser Ala Thr Asn Gln Val Pro Glu Ala Ser Leu
                325                 330                 335

Ala His His Pro Gln Ala Gly Leu Val Gln Pro Asn Ile Gly Gln Ser
            340                 345                 350

Pro Ala Gln Gly Ala Ala Pro Ala Asp Ser Trp Ser Pro Glu Phe Asp
        355                 360                 365

Leu Val Gly Cys Glu Thr Asp Ser Gly Glu Cys Phe Asp Pro Ile Met
    370                 375                 380

Ala Val Leu Asp Glu Ser Glu Gly Asp Ala Ile Ser Pro Glu Gly Glu
385                 390                 395                 400

Gly Lys Met Asn Glu Leu Leu Glu Gly Val Pro Lys Leu Pro Gly Ile
                405                 410                 415

Gln Asp Pro Phe Trp Glu Gln Phe Phe Ser Val Glu Leu Pro Ala Ile
            420                 425                 430

Ala Asp Thr Asp Asp Ile Leu Ser Gly Ser Val Glu Asn Asn Asp Leu
        435                 440                 445

Val Leu Glu Gln Glu Pro Asn Glu Trp Thr Arg Asn Glu Gln Gln Met
    450                 455                 460

Lys Tyr Leu Thr Glu Gln Met Gly Leu Leu Ser Ser Glu Ala Gln Arg
465                 470                 475                 480

Lys

<210>  2
<211>  497
<212>  PRT
<213>  Zea mays

<400>  2

Met Gln Gly Gly Gly Val Ala Ala Ala Ala Val Pro Val His Ala Ala
1               5                   10                  15

Val Gly Gly Asp Gly Gly Gly Ala Ala Ala Ala Pro Pro Pro Pro Phe
            20                  25                  30

Leu Met Lys Thr Tyr Glu Met Val Asp Asp Pro Ala Thr Asp Gly Val
        35                  40                  45

Val Ser Trp Gly Pro Gly Asn Asn Ser Phe Ile Val Trp Asn Thr Pro
    50                  55                  60

Glu Phe Ala Arg Asp Leu Leu Pro Lys Tyr Phe Lys His Ser Asn Phe
65                  70                  75                  80

Ser Ser Phe Val Arg Gln Leu Asn Thr Tyr Gly Phe Arg Lys Val Asp
                85                  90                  95

Pro Asp Arg Trp Glu Phe Ala Asn Glu Gly Phe Leu Arg Gly Gln Lys
            100                 105                 110

His Leu Leu Lys Thr Ile Asn Arg Arg Lys Pro Ser Leu Gln Gly Asn
        115                 120                 125

Ser Gln Pro Gln Gln Pro Gln Leu Gln Asn Ala Pro Val Pro Ser Cys
    130                 135                 140

Val Glu Val Gly Lys Phe Gly Leu Glu Glu Glu Ile Glu Arg Leu Lys
145                 150                 155                 160

Arg Asp Lys Asn Val Leu Met Gln Glu Leu Val Arg Leu Arg Gln Gln
                165                 170                 175

Gln Gln Thr Thr Asp His Gln Leu Gln Thr Leu Gly Lys Arg Leu Gln

            180                 185                 190

Gly Met Glu Ser Arg Gln Gln Gln Met Met Ser Phe Leu Ala Lys Ala
        195                 200                 205

Met Gln Ser Pro Gly Phe Leu Ala Gln Phe Val Gln Arg Asn Glu Asn
    210                 215                 220

Ser Arg Arg Arg Ile Val Ala Ala Asn Lys Lys Arg Arg Leu Pro Lys
225                 230                 235                 240

Gln Asp Gly Gly Leu Glu Ser Glu Ser Ser Ala Ala Ser Leu Asp Gly
                245                 250                 255

Gln Ile Ile Lys Tyr Gln Pro Ser Ile Asn Glu Ala Ala Lys Ala Met
            260                 265                 270

Leu Arg Lys Ile Leu Asn Val Asp Ser Ser His Arg Phe Glu Ser Met
        275                 280                 285

Gly Asn Ser Asp Asn Ser Asn Asn Asn Asn Ile Leu Leu Glu Asp Tyr
    290                 295                 300

Met Pro Ala Ala Gln Ala Phe Glu Ser Ser Ser Ser Thr Arg Asn Ser
305                 310                 315                 320

Gly Val Thr Leu Ala Glu Val Pro Ala Asn Ser Gly Leu Ala Tyr Val
                325                 330                 335

Gly Thr Ser Ser Ala Ile Cys Ser Pro Pro Ala Ala Pro Glu Met Gln
            340                 345                 350

Cys Pro Val Val Leu Asp Asn Lys Ala Tyr Lys Gln Val Ala Ser Met
        355                 360                 365

Ser Ala Val Pro Pro Val Ser Ser Asp Met Gly Ile Ile Pro Glu Phe
    370                 375                 380

Ser Asp Leu Ala Asp Leu Val Ser Val Asp Ile Leu Gly Gly Ala Phe
385                 390                 395                 400

Glu Met Met Pro Gly Pro Glu Phe Pro Leu Pro Glu Glu Gly Asp Asp
                405                 410                 415

Gly Thr Thr Met Tyr Asn Asn Asn Asp Glu Glu Ala Gln Ser Leu Pro
            420                 425                 430

Gly Ile Ile Asn Ser Ile Trp Glu Gln Phe Leu Val Gly Ser Pro Leu
        435                 440                 445

Ser Thr Asp Asn Glu Glu Val Asp Ser Ala Gly Gly Leu Tyr Ala Gln
    450                 455                 460

Glu Asp Gly Trp Ser Lys Val Gly Asn Ile Ala Asn Leu Thr Glu Gln
465                 470                 475                 480

Met Gly Leu Val Ser Ser Thr Asn His Arg Asp Ser Gly Asn Gly Leu
                485                 490                 495

Tyr

<210>  3
<211>  357
<212>  PRT
<213>  Oryza sativa

<400>  3

Met Asn Tyr Arg Val Val Asn Pro Val Lys Val Glu Ser Gly Pro Ser
1               5                   10                  15

Thr Gly Val Ala Asn Gly Gln Pro Pro Arg Pro Met Asp Gly Leu Ala
            20                  25                  30

Asp Gly Gly Pro Pro Pro Phe Leu Thr Lys Thr Tyr Asp Met Val Asp
        35                  40                  45

Asp Pro Thr Thr Asp Ala Val Val Ser Trp Ser Ala Thr Asn Asn Ser
    50                  55                  60

Phe Val Val Trp Asp Pro His Leu Phe Gly Asn Val Leu Leu Pro Arg
65                  70                  75                  80

Tyr Phe Lys His Asn Asn Phe Ser Ser Phe Val Arg Gln Leu Asn Thr
                85                  90                  95

Tyr Gly Phe Arg Lys Val Asp Pro Asp Lys Trp Glu Phe Ala Asn Glu
            100                 105                 110

Gly Phe Leu Arg Gly Gln Lys His Leu Leu Lys Ser Ile Lys Arg Arg
        115                 120                 125

Lys Pro Pro Asn Ser Ser Pro Ser Gln Gln Ser Leu Gly Ser Phe Leu
    130                 135                 140

Glu Val Gly His Phe Gly Tyr Glu Gly Glu Ile Asp Gln Leu Lys Arg
145                 150                 155                 160

Asp Lys His Leu Leu Met Ala Glu Val Val Lys Leu Arg Gln Glu Gln
                165                 170                 175

Gln Asn Thr Lys Ser Asp Leu Gln Ala Met Glu Gln Lys Leu Gln Gly
            180                 185                 190

Thr Glu Gln Lys Gln Gln His Met Met Ala Phe Leu Ser Arg Val Met
        195                 200                 205

His Asn Pro Glu Phe Ile Arg Gln Leu Phe Ser Gln Ser Glu Met Arg
    210                 215                 220

Lys Glu Leu Glu Glu Phe Val Ser Lys Lys Arg Arg Arg Arg Ile Asp
225                 230                 235                 240

Gln Gly Pro Glu Leu Asp Ser Met Gly Thr Gly Ser Ser Pro Glu Gln
                245                 250                 255

Val Ser Gln Val Met Phe Glu Pro His Asp Pro Val Asp Ser Leu Phe
            260                 265                 270

Asn Gly Val Pro Ser Asp Leu Glu Ser Ser Ser Val Glu Ala Asn Gly
        275                 280                 285

Gly Lys Ala Gln Gln Asp Val Ala Ser Ser Ser Ser Glu His Gly Lys
    290                 295                 300

Ile Lys Pro Ser Asn Gly Glu Leu Asn Glu Asp Phe Trp Glu Asp Leu
305                 310                 315                 320

Leu His Glu Gly Gly Leu Asp Glu Asp Thr Arg Asn Pro Ala Ile Asp
                325                 330                 335

Asp Met Asn Leu Leu Ser Gln Lys Met Gly Tyr Leu Asn Ser Ser Ser
            340                 345                 350

Thr Lys Ser Pro Gln
        355

<210>  4
<211>  358
<212>  PRT
<213>  Oryza sativa

<400>  4
   Met Asp Pro Ala Ala Ala Gly Ile Val Lys Glu Glu Met Leu Glu Ser
   1               5                   10                  15
   Gln Gln Gln Gln Arg Gln Glu Asp Gly Gly Ala Ala Pro Arg Pro Met
               20                  25                  30
   Glu Gly Leu His Glu Val Gly Pro Pro Pro Phe Leu Thr Lys Thr Tyr
           35                  40                  45
   Asp Leu Val Glu Asp Pro Ala Thr Asp Gly Val Val Ser Trp Ser Arg
       50                  55                  60
   Ala Gly Asn Ser Phe Val Val Trp Asp Pro His Val Phe Ala Asp Leu
   65                  70                  75                  80
   Leu Leu Pro Arg Leu Phe Lys His Asn Asn Phe Ser Ser Phe Val Arg
                   85                  90                  95
   Gln Leu Asn Thr Tyr Gly Phe Arg Lys Val Asp Pro Asp Arg Trp Glu
               100                 105                 110
   Phe Ala Asn Glu Gly Phe Leu Arg Gly Gln Arg His Leu Leu Lys Thr
           115                 120                 125
   Ile Lys Arg Arg Lys Pro Pro Ser Asn Ala Pro Pro Ser Gln Gln Gln
       130                 135                 140
   Ser Leu Thr Ser Cys Leu Glu Val Gly Glu Phe Gly Phe Glu Glu Glu
   145                 150                 155                 160
   Ile Asp Arg Leu Lys Arg Asp Lys Asn Ile Leu Ile Thr Glu Val Val
                   165                 170                 175
   Lys Leu Arg Gln Glu Gln Gln Ala Thr Lys Asp His Val Lys Ala Met
               180                 185                 190
   Glu Asp Arg Leu Arg Ala Ala Glu Gln Lys Gln Val Gln Met Met Gly
           195                 200                 205
   Phe Leu Ala Arg Ala Met Arg Asn Pro Glu Phe Phe Gln Gln Leu Ala
       210                 215                 220
   Gln Gln Lys Glu Lys Arg Lys Glu Leu Glu Asp Ala Ile Ser Lys Lys
   225                 230                 235                 240
   Arg Arg Arg Pro Ile Asp Asn Val Pro Phe Tyr Asp Pro Gly Glu Thr
                   245                 250                 255
   Ser Gln Thr Glu Gln Leu Asp Ser Pro Tyr Leu Phe Asp Ser Gly Val
               260                 265                 270
   Leu Asn Glu Leu Ser Glu Pro Gly Ile Pro Glu Leu Glu Asn Leu Ala
           275                 280                 285
   Val Asn Ile Gln Asp Leu Gly Lys Gly Lys Val Asp Glu Glu Arg Gln
       290                 295                 300
   Asn Gln Thr Asn Gly Gln Ala Glu Leu Gly Asp Asp Phe Trp Ala Glu
   305                 310                 315                 320
   Leu Leu Val Glu Asp Phe Thr Gly Lys Glu Glu Gln Ser Glu Leu Asp
                   325                 330                 335
   Gly Lys Ile Asp Gly Ile Asp Glu Leu Ala Gln Gln Leu Gly Tyr Leu
               340                 345                 350
   Ser Ser Thr Ser Pro Lys
           355

<210>  5
<211>  464
<212>  PRT
<213>  Glycine max

<400>  5

Met Glu Gly Ala Ser Trp Asn Ser Ser Val Cys Val Ala Pro Phe Leu
1               5                   10                  15

Ser Lys Thr Tyr Asp Met Val Asp Asp Pro Ser Thr Asp Leu Val Val
            20                  25                  30

Ser Trp Gly Glu Asn Asn Asn Ser Phe Val Val Trp Asn Val Pro Gln
        35                  40                  45

Phe Ala Thr Asp Ile Leu Pro Asn His Phe Lys His Asn Asn Phe Ser
    50                  55                  60

Ser Phe Val Arg Gln Leu Asn Thr Tyr Gly Phe Arg Lys Val Asp Pro
65                  70                  75                  80

Asp Arg Trp Glu Phe Ala Asn Glu Gly Phe Leu Arg Gly Glu Lys Gln
                85                  90                  95

Leu Leu Lys Ser Ile Ser Arg Arg Lys Ser Ala His Val Asn Gly Ser
            100                 105                 110

Gln Gln Ala Ser Gln Val His Lys Ser Ala Ala Gly Ala Cys Val Glu
        115                 120                 125

Val Gly Lys Phe Gly Leu Glu Glu Glu Val Glu Arg Leu Lys Arg Asp
    130                 135                 140

Lys Asn Val Leu Met Gln Glu Leu Val Arg Leu Arg Gln Lys Gln Gln

145                 150                 155                 160

Gly Thr Asp Asn Gln Leu Gln Asn Val Gly Gln Arg Val Gln Ser Met
                165                 170                 175

Glu Gln Arg Gln Gln Gln Met Met Ser Phe Leu Ala Lys Ala Met Gln
            180                 185                 190

Ser Pro Gly Phe Leu Ala Gln Phe Val Gln Gln Gln Asn Glu Ser Ser
        195                 200                 205

Lys His Ile Pro Gly Ser Asn Lys Lys Arg Arg Leu Gln Arg Gln Glu
    210                 215                 220

Glu Asp Ser Leu Ala Thr Lys Asp Leu His Ser Ser Leu Asp Gly His
225                 230                 235                 240

Ile Val Lys Tyr Gln Ser Ser Ile Asn Glu Ala Ala Lys Ala Leu Phe
                245                 250                 255

Arg Gln Ile Leu Gln Ile Asn Asn Ser Thr Thr Gln Ser Ser Ile Lys
            260                 265                 270

Asn Pro Asp Val Phe Leu Ile Asp Asp Ile Pro Ser Ala Ile Ala Ser
        275                 280                 285

Asp Ser Ser Ser Ser Ser Thr Gln Val Ser Asn Val Met Val Pro Pro
    290                 295                 300

Ile Ser Glu Leu Thr Ser Met Glu Val Gly Ser Gln Phe Pro Leu Ser
305                 310                 315                 320

Cys Met Pro Ser Asn Ile Ser Glu Val Gln Ser Ser Pro Ala Val Leu
                325                 330                 335

Gly Phe Cys Arg Ser Gln Gly Met Glu Thr Glu Ser Ser Leu Leu Asn
            340                 345                 350

His Glu Leu Asn Val Met Gly Ala Asp Thr Arg Asn Met Gly Glu Ile
        355                 360                 365

Asp Met Met Ser Thr Val Leu Asp Glu Ala Asp Glu Ala Asp His Phe
    370                 375                 380

Ser Pro Asp Thr Asp Gly Ile Ser Lys Leu Pro Glu Ile Ser Asp Glu
385                 390                 395                 400

Phe Trp Glu Leu Phe Leu Arg Pro Ser Pro Leu Thr Glu Asp Thr Glu
                405                 410                 415

Glu Thr Lys Cys Ser Ser Leu Gly Cys Gly Leu Thr Glu Asp Gln Gly
            420                 425                 430

Leu Pro Ser Glu Met Gly Asn Lys Gln Glu Asn Phe Asp Thr Ile Gln
        435                 440                 445

His Val Asp Cys Leu Thr Gln Gln Met Gly Leu His Ala Leu Glu Tyr
    450                 455                 460

<210>  6
<211>  527
<212>  PRT
<213>  Sorghum bicolor

<400>  6

Met Gln Gly Gly Val Val Ala His Ala Ala Ala Ala Ala Ala Ala Ala
1               5                   10                  15

Ser Thr Val Thr Thr Ala Val Ala Pro Pro Val Pro Ala His Ala Ala
            20                  25                  30

Val Val Gly Asn Gly Gly Gly Pro Ala Ala Ala Ala Pro Pro Pro Pro
        35                  40                  45

Phe Leu Met Lys Thr Tyr Glu Met Val Asp Asp Pro Ala Thr Asp Asp
    50                  55                  60

Val Val Ser Trp Gly Pro Gly Asn Asn Ser Phe Ile Val Trp Asn Thr
65                  70                  75                  80

Pro Glu Phe Ala Arg Asp Leu Leu Pro Lys Tyr Phe Lys His Ser Asn
                85                  90                  95

Phe Ser Ser Phe Val Arg Gln Leu Asn Thr Tyr Gly Phe Arg Lys Val
            100                 105                 110

Asp Pro Asp Arg Trp Glu Phe Ala Asn Glu Gly Phe Leu Arg Gly Gln
        115                 120                 125

Lys His Leu Leu Lys Thr Ile Asn Arg Arg Lys Pro Ser Leu Gln Gly
    130                 135                 140

Asn Ser Gln Pro Gln Gln Pro Gln Leu Gln Asn Ala Pro Val Pro Ser
145                 150                 155                 160

Cys Val Glu Val Gly Lys Phe Gly Leu Glu Glu Glu Ile Glu Arg Leu
                165                 170                 175

Lys Arg Asp Lys Asn Val Leu Met Gln Glu Leu Val Arg Leu Arg Gln
            180                 185                 190

Gln Gln Gln Thr Thr Asp His Gln Leu Gln Thr Leu Gly Lys Arg Leu
        195                 200                 205

Gln Gly Met Glu Ser Arg Gln Gln Gln Met Met Ser Phe Leu Ala Lys
    210                 215                 220

Ala Met Gln Ser Pro Gly Phe Leu Ala Gln Phe Val Gln Gln Asn Glu
225                 230                 235                 240

Asn Ser Arg Arg Arg Ile Val Ala Ala Asn Lys Lys Arg Arg Leu Pro
                245                 250                 255

Lys Gln Asp Gly Gly Leu Asp Ser Glu Ser Ala Ala Ala Ser Leu Asp
            260                 265                 270

Gly Gln Ile Ile Lys Tyr Gln Pro Leu Ile Asn Glu Ala Ala Lys Ala
        275                 280                 285

Met Leu Arg Lys Ile Leu Lys Leu Asp Ser Ser His Arg Phe Glu Ser
    290                 295                 300

Met Gly Asn Ser Asp Asn Asn Asn Phe Leu Leu Glu Asn Tyr Met Pro
305                 310                 315                 320

Ala Ala Gln Ala Phe Glu Ser Ser Ser Ser Thr Arg Asn Ser Gly Val
                325                 330                 335

Thr Leu Ala Glu Val Pro Ala Asn Ser Gly Leu Pro Tyr Val Ser Ala
            340                 345                 350

Ser Ser Gly Leu Ser Ala Ile Cys Ser Pro Ser Val Ala Pro Glu Ile
        355                 360                 365

Gln Cys Pro Val Val Leu Asp Asn Lys Leu Ser Asn Gln Val Pro Asn
    370                 375                 380

Met Ser Ala Val Pro Pro Val Ser Asn Pro Ile Thr Ala Gly Ser Ser
385                 390                 395                 400

Asp Ile Ser Ile Pro Glu Phe Ser Asp Leu Ala Asp Leu Val Asn Glu
                405                 410                 415

Asp Ser Val Asn Ile Pro Gly Gly Ala Phe Glu Met Pro Gly Pro Glu
            420                 425                 430

Phe Pro Leu Pro Glu Gly Asp Asp Ser Val Pro Ile Glu Thr Asp Glu

        435                 440                 445

Thr Met Tyr Asn Asn Asp Glu Thr Gln Ser Leu Pro Gly Ile Ile Asp
    450                 455                 460

Ser Phe Trp Glu Gln Phe Leu Val Gly Ser Pro Leu Ser Ala Asp Asn
465                 470                 475                 480

Asp Glu Val Asp Ser Gly Gly Leu Asp Ala Arg Gly Ser Pro Gln Glu
                485                 490                 495

Asn Gly Trp Ser Lys Val Gly Asn Ile Ser Asn Leu Thr Glu Gln Met
            500                 505                 510

Gly Leu Leu Ser Ser Thr Asn His Arg Asp Ser Gly Asn Gly Leu
        515                 520                 525

<210>  7
<211>  510
<212>  PRT
<213>  Hordeum vulgare

<400>  7

Met Glu Gly Gly Val Ala Leu Ala Ser Ser Val Thr Thr Ala Val Ala
1               5                   10                  15

Pro Pro Gly Gln Gly Ala Gly Ala Pro Pro Pro Phe Leu Met Lys Thr
            20                  25                  30

Tyr Asp Met Val Asp Asp Pro Ala Thr Asp Ala Val Val Ser Trp Gly
        35                  40                  45

Pro Ala Ser Asn Ser Phe Ile Val Trp Asn Thr Pro Glu Phe Ala Arg
    50                  55                  60

Asp Leu Leu Pro Lys Tyr Phe Lys His Asn Asn Phe Ser Ser Phe Val
65                  70                  75                  80

Arg Gln Leu Asn Thr Tyr Gly Phe Arg Lys Val Asp Pro Asp Lys Trp
                85                  90                  95

Glu Phe Ala Asn Glu Gly Phe Leu Arg Gly Gln Lys His Leu Leu Lys
            100                 105                 110

Thr Ile Asn Arg Arg Lys Pro Leu His Ala Asn Asn Gln Val Gln Val
        115                 120                 125

Gln Gln Gln Gln His Gln Gln Gln His Gln Gln Gln Pro Gln Leu Gln
    130                 135                 140

Asn Ala Pro Ile Pro Ser Cys Val Glu Val Gly Lys Phe Gly Met Glu
145                 150                 155                 160

Glu Glu Ile Glu Met Leu Lys Arg Asp Lys Asn Val Leu Met Gln Glu
                165                 170                 175

Leu Val Arg Leu Arg Gln Gln Gln Gln Thr Thr Asp His Gln Leu Gln
            180                 185                 190

Thr Leu Gly Lys Arg Leu His Gly Met Glu Gln Arg Gln Gln Gln Met
        195                 200                 205

Met Ser Phe Leu Ala Lys Ala Met Gln Ser Pro Gly Phe Leu Ala Gln
    210                 215                 220

Phe Val Gln Gln Asn Glu Asn Ser Lys Arg Arg Ile Val Ala Ala Asn
225                 230                 235                 240

Lys Lys Arg Arg Leu Pro Lys Gln Asp Asp Gly Leu Asn Pro Glu Ser
                245                 250                 255

Ala Leu Leu Asp Gly Gln Ile Ile Lys Tyr Gln Pro Met Ile Asn Glu
            260                 265                 270

Ala Ala Lys Ala Met Leu Arg Lys Ile Leu Gln Gln Asp Thr Ser Pro
        275                 280                 285

His Arg Phe Glu Ser Met Gly Asn Ser Asp Asn Leu Leu Leu Glu Asn
    290                 295                 300

Cys Met Pro Ser Ala Gln Thr Phe Asp Ser Ser Ser Thr Arg Asn
305                 310                 315                 320

Ser Ala Val Thr Leu Ala Glu Val Pro Gly Asn Ser Gly Met Pro Tyr
                325                 330                 335

Met Pro Thr Ser Ser Gly Leu Ser Ala Ile Cys Ser Ser Ser Ser Pro
            340                 345                 350

Pro Glu Met Gln Cys Pro Pro Val Leu Asp Ser Asn Ser Ser Thr Gln
        355                 360                 365

Leu Pro Asn Met Ser Ala Val Pro Ser Val Pro Lys Ala Met Thr Pro
    370                 375                 380

Gly Leu Ser Asp Ile Ser Ile Pro Gly Phe Pro Asp Leu His Asp Leu
385                 390                 395                 400

Ile Thr Glu Asp Ala Ile Asn Ile Pro Val Glu Asn Tyr Ala Met Pro
                405                 410                 415

Gly Pro Glu Cys Ile Phe Pro Leu Pro Glu Gly Ser Asp Asp Ser Val
            420                 425                 430

Pro Met Asp Pro Ile Asp Thr Asp Glu Ile Asp Asp Thr Gln Lys Leu
        435                 440                 445

Pro Gly Ile Ile Asp Ser Phe Trp Glu Gln Phe Leu Cys Ala Ser Pro
    450                 455                 460

Leu Ser Val Asp Asn Asp Glu Val Asp Ser Gly Leu Leu Asp Thr Arg
465                 470                 475                 480

Glu Ala Gln Glu Glu Asn Gly Trp Thr Arg Thr Glu Asn Leu Ala Asn
                485                 490                 495

Leu Thr Glu Gln Met Gly Leu Leu Ser Ser Asn His Arg Gly
            500                 505                 510

<210>  8
<211>  418
<212>  DNA
<213>  Triticum aestivum

<400>  8
agctttgagt ggccgtagat ttgcaaaagc aatggctaac agacacatat tctgccaaac      60

cccaagaagg ataatcactt ttcttagata aaaaagaaca gaccaatata caaacatcca     120

cacttctgca aacaatacat cagaactagg attacgccga ttacgtggct ttagcagact     180

gtccaaaaat ctgttttgca aagctccaat tgctccttgc ttatccagct tcttttgtgt     240

tggcaaactg cgcttttcca accgattttg ttcttctcgc gctttcttct tagcctaaac     300

aaacctcacc gtgcacgcag ccatggtcct gaaccttcac ctcgtcccta taaaagccta     360

gccaaccttc acaatcttat catcacccac aacaccgagc accacaaact agagatcc       418

<210>  9
<211>  2461
<212>  DNA
<213>  Artificial

<220>
<223>  Construct pHMWG - AtHSF3

<220>
<221>  promoter
<222>  (1)..(418)
<223>  promoter HMWG

<220>
<221>  gene

<222>   (468)..(1910)
<223>   AtHSF3 coding sequence

<400>   9
agctttgagt ggccgtagat ttgcaaaagc aatggctaac agacacatat tctgccaaac      60

cccaagaagg ataatcactt ttcttagata aaaaagaaca gaccaatata caaacatcca     120

cacttctgca aacaatacat cagaactagg attacgccga ttacgtggct ttagcagact     180

gtccaaaaat ctgttttgca aagctccaat tgctccttgc ttatccagct tcttttgtgt     240

tggcaaactg cgcttttcca accgattttg ttcttctcgc gctttcttct tagcctaaac     300

aaacctcacc gtgcacgcag ccatggtcct gaaccttcac ctcgtcccta taaaagccta     360

gccaaccttc acaatcttat catcacccac aacaccgagc accacaaact agagatccga     420

attcgattca ggatatcaca agtttgtaca aaaaagcagg ctccaccatg gagtccgtgc     480

ctgagtccgt gccaagccct aactccaaca ccccctctat cccgccgcca gtgaacagcg     540

tgcccccctt cctgtccaag acctacgaca tggtcgacga cccgctgacc aacgaggtgg     600

tctcttggtc cagcggcaac aactccttcg tggtgtggtc tgcgcccgag ttctccaagg     660

tgctgctgcc aaagtacttc aagcacaaca acttcagctc tttcgtgagg cagctcaaca     720

cctacggctt caggaaggtg gaccccgata ggtgggagtt cgcgaacgag ggcttcctcc     780

gcggccgcaa gcagctcctg aagagcatcg tcaggaggaa gccgagccac gtccagcaga     840

accagcagca gacccaggtg cagtcttcct ctgtcggcgc gtgcgtggag gtgggcaagt     900

tcggcatcga ggaggaggtc gagcgcctca agagggataa gaacgtcctg atgcaggagc     960

tcgtcaggct gaggcagcag cagcaggcca ccgagaacca gctgcagaac gtcggccaga    1020

aggtccaggt gatggagcag aggcagcagc agatgatgag cttcctggcc aaggccgtcc    1080

agtcccctgg cttcctgaac cagctcgtgc agcagaacaa caacgacggc aacaggcaga    1140

tccctggcag caacaagaag cgccgcctcc ctgtcgacga gcaggagaac cgcggcgaca    1200

acgtggccaa cggcctgaac aggcagatcg tgaggtacca gcctagcatc aacgaggccg    1260

cgcagaacat gctccgccag ttcctcaaca cttccacctc tccaaggtac gagtccgtct    1320

ccaacaaccc tgacagcttc ctgctcggcg acgtgccctc tagcacctcc gtggacaacg    1380

gcaacccgag ctccagggtg tctggcgtca ccctcgctga gttctctccc aacaccgtcc    1440

agagcgccac caaccaggtg ccggaggcgt ctctcgccca ccacccccag gccggcctcg    1500

tgcagccaaa catcggccag agccccgccc agggcgccgc cccggctgac tcctggtccc    1560

cagagttcga cctggtgggc tgcgagaccg atagcggcga gtgcttcgac ccaatcatgg    1620

cggtgctgga tgagtccgag ggcgacgcga tctctcccga gggcgagggc aagatgaacg    1680

agctgctgga gggcgtccca aagctcccgg gcatccagga ccccttctgg gagcagttct    1740

tcagcgtgga gctccccgcc atcgctgata ccgatgacat cctgagcggc tctgtcgaga    1800

acaacgacct cgtgctcgag caggagccta acgagtggac ccgcaacgag cagcagatga    1860

agtacctgac cgagcagatg ggcctcctca gcagcgaggc ccagcgcaag taatctagac 1920

ccagctttct tgtacaaagt ggtgatatcc taaatgctct taactgagct aattatgtaa 1980

tgcacataca catatttaca tagatatgca tatttatata tagcatgtat attgtactac 2040

atgcattgct tcttaataca tgtagtaaag atatatgcaa aaatagtcga aagatttgtt 2100

tacatataaa atcaccaata tttattgtta ttgtattttc atgaataaag taataagatt 2160

atttgtctaa tattttgatt tactagtact agaaatgaaa aggaatatgc acaatttcag 2220

cattatagtt tggtaggcaa aatggagtga gaatagagtt tcatagtata tactaaggtt 2280

cttaattgtg caaatagttg atacaagtca catgggccaa gtttgtaaat cttaaatcga 2340

aatatgcctt cttctttttt tgcatgaaaa tgctagtaat ttataagtgt gtttttcaat 2400

aagagatgct aaataccaaa attaacctag ttttcagtga gcgcttgcat tattgtggca 2460

t 2461